# EUROPÄISCHE PATENTSCHRIFT

(11) **EP 0 878 467 B1**
(45) Veröffentlichungstag und Bekanntmachung des Hinweises auf die Patenterteilung: **31.10.2001**
(21) Anmeldenummer: 98108039.3
(22) Anmeldetag: 02.05.1998
(51) Int. Cl.: C07D 217/26, A61K 31/47

(54) **Substituierte 6- und 7-Aminotetrahydroisochinolincarbonsäuren**
Substituted 6- and 7-aminotetrahydroisoquinolinecarboxylic acids
Acids 6- et 7-aminotetrahydroisoquinolinecarboxyliques substitués

(30) Priorität: 13.05.1997 DE 19719817
(43) Veröffentlichungstag der Anmeldung: 18.11.1998
(73) Patentinhaber: HOECHST AKTIENGESELLSCHAFT, 65929 Frankfurt am Main (DE)
(72) Erfinder: Schudok, Manfred, Dr., 65817 Eppstein/Ts. (DE)

(56) Entgegenhaltungen:
- EP-A- 0 606 046
- EP-A- 0 614 911
- WO-A-95/29892
- WO-A-96/00214
- WO-A-96/33172
- WO-A-97/18194
- WO-A-97/49674

## Beschreibung

Die Erfindung betrifft neue substituierte 6- und 7-Aminotetrahydroisochinolincarbonsäuren, Verfahren zu ihrer Herstellung und Verwendung derselben als Arzneimittel.

In den Anmeldungen EP 0 606 046, WO 95/35276 und WO 96/27583 werden Arylsulfonylaminohydroxamsäuren und deren Wirkung als Matrix-Metalloproteinase-Inhibitoren beschrieben. Spezielle Arylsulfonylaminocarbonsäuren dienen als Zwischenprodukte zur Darstellung von Thrombin-Inhibitoren (EP 0 468 231) und Aldose-Reduktase-Inhibitoren (EP 0 305 947).

In den Anmeldungen EP0 757 037 und WO 97/18194 wird die Wirkung von Sulfonylaminosäure - Derivaten bzw. (hetero) cyclischen α-IMINOHYDROXAM - und Carbonsäure- Derivaten als Metallproteinase- Inhibitoren beschrieben.

In dem Bestreben wirksame Verbindungen zur Behandlung von Bindegewebserkrankungen zu finden, wurde nun gefunden, daß die erfindungsgemäßen Carbonsäuren starke Inhibitoren der Matrix-Metalloproteinasen sind. Dabei wird auf die Hemmung von Stromelysin (Matrix Metalloproteinase 3) und der Neutrophilen Kollagenase (MMP-8) besonderer Wert gelegt, da beide Enzyme insbesondere beim Abbau der Proteoglykane, als wichtige Bestandteile des Knorpelgewebes, maßgeblich beteiligt sind (A. J. Fosang et al. J. Clin. Invest. 98 (1996) 2292-2299).

Die Erfindung betrifft daher die Verbindung der Formel I 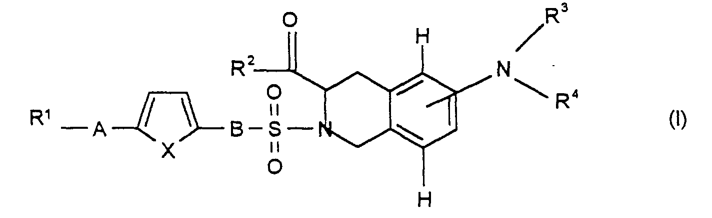 und/oder eine stereoisomere Form der Verbindung der Formel I und/oder ein physiologisch verträgliches Salz der Verbindung der Formel I, wobei R¹ für
1. Phenyl,
2. Phenyl, welches ein- oder zweifach substituiert ist durch
   2.1. (C₁-C₆)-Alkyl, gerade, cyclisch oder verzweigt,
   2.2. -OH,
   2.3. (C₁-C₆)-Alkyl-C(O)-O-,
   2.4. (C₁-C₆)-Alkyl-O-,
   2.5. (C₁-C₆)-Alkyl-O-(C₁-C₄)-Alkyl-O-,
   2.6. Halogen,
   2.7. -CF₃,
   2.8. -CN,
   2.9. -NO₂,
   2.10. HO-C(O)-,
   2.11. (C₁-C₆)-Alkyl-O-C(O)-,
   2.12. Methylendioxo,
   2.13. R⁵-(R⁶ )N-C(O)-, worin R⁵ und R⁶ gleich oder verschieden sind und für Wasserstoffatom oder (C₁-C₆)-Alkyl- stehen, oder
   2.14. R⁵-(R⁶)N-, worin R⁵ und R⁶ gleich oder verschieden sind und für Wasserstoffatom oder (C₁-C₆)-Alkyl- stehen,
3. einen Heteroaromaten aus der nachfolgenden Gruppe 3.1. bis 3.15., der unsubstituiert oder wie unter 2.1 bis 2.14 beschrieben substituiert ist,
   3.1. Pyrrol,
   3.2. Pyrazol,
   3.3. Imidazol,
   3.4. Triazol,
   3.5. Thiophen,
   3.6. Thiazol,
   3.7. Oxazol,
   3.8. Isoxazol,
   3.9. Pyridin,
   3.10. Pyrimidin,
   3.11. Indol,
   3.12 Benzothiophen,
   3.13. Benzimidazol,
   3.14. Benzoxazol oder
   3.15. Benzothiazol,
4. -OH steht und A eine kovalente Bindung bedeutet,
5. -O-R¹⁴ steht und A eine kovalente Bindung, -CH=CH- oder -C≡C- bedeutet und worin R¹⁴
   1) (C₁-C₆)-Alkyl,
   2) (C₃-C₆)-Cycloalkyl,
   3) Benzyl oder
   4) Phenyl bedeutet,
6. -COOH steht und A eine kovalente Bindung, -CH=CH- oder -C≡C- bedeutet,
7. (C₁-C₆)-Alkyl,
8. (C₃-C₆)-Cycloalkyl-O-(C₁-C₄)-alkyl,
9. Halogen steht und A eine kovalente Bindung, -CH=CH- oder -C≡C- bedeutet,
10. -CN steht und A eine kovalente Bindung, -CH=CH- oder -C≡C- bedeutet,
11. -NO₂ steht und A eine kovalente Bindung, -CH=CH- oder -C≡C- bedeutet, oder
12. -CF₃ steht, und
R² für
1. HO(H)N- oder
2. R⁷-O- steht, worin R⁷
   2.1 Wasserstoffatom,
   2.2 (C₁-C₆)-Alkyl,
   2.3 Allyl oder
   2.4 Benzyl bedeutet,
R³ und R⁴ gleich oder verschieden sind und für
1. Wasserstoffatom,
2. (C₁-C₆)-Alkyl,
3. Phenyl-(CH₂)ₘ, worin Phenyl unsubstituiert oder ein- oder zweifach wie unter 2.1. bis 2.14. beschrieben substituiert ist und m die ganze Zahl Null, 1, 2 oder 3 bedeutet,
4. R⁸-(CO)-, worin R⁸
   4.1 (C₁-C₈)-Alkyl,
   4.2 Phenyl-(CH₂)ₘ-, worin Phenyl unsubstituiert oder ein- oder zweifach wie unter 2.1. bis 2.14. beschrieben substituiert ist und m die ganze Zahl Null, 1, 2 oder 3 darstellt,
   4.3 R⁷-O-C(O)-(CH₂)ₙ-, worin R⁷ wie oben definiert ist und n die ganze Zahl Null, 1, 2, 3, 4, 5 oder 6 bedeutet,
   4.4 R⁷-N(H)-(R⁹)-C(H)-, worin R⁷ wie oben definiert ist und R⁹ den charakteristischen Rest einer proteinogenen α-Aminosäure bedeutet und worin R⁹ unsubstituiert oder an einem Sauerstoff- oder Schwefelatom ein- oder zweifach durch (C₁-C₄)-Alkyl, Benzyl, Allyl substituiert ist oder durch eine N-Schutzgruppe substituiert ist,
   4.5 R⁷-C(O)-N(H)-(R⁹)-C(H)-, worin R⁷ und R⁹ wie unter 4.4 definiert sind, oder
   4.6 R¹⁰-O-C(O)-N(H)-(R⁹)-C(H)-, R⁹ wie unter 4.4 definiert ist und worin R¹⁰
      4.6.1 (C₁-C₆)-Alkyl,
      4.6.2 Allyl,
      4.6.3 Benzyl oder
      4.6.4 (9-Fluorenyl)-methyl bedeutet,
5. R¹⁰-O-C(O)-, worin R¹⁰ wie unter 4.6.1 bis 4.6.4 definiert ist,
6. R¹⁵-SO₂-, worin R¹⁵
   6.1 (C₁-C₆)-Alkyl,
   6.2 Allyl oder
   6.3 Phenyl-(CH₂)ₘ-, worin Phenyl unsubstituiert oder ein- oder zweifach wie unter 2.1 bis 2.14 beschrieben substituiert ist und m die ganze Zahl Null, 1, 2 oder 3 darstellt, oder
7. H₂N-C(=NH)- stehen, oder
R³ und R⁴ zusammen mit dem N-Atom einen Rest der Formel Xₐ oder X_{b} bilden, 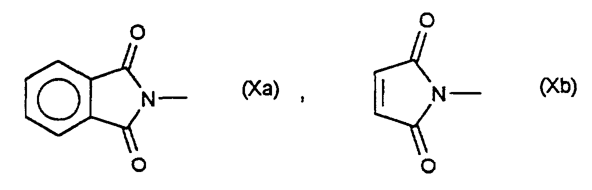 A für
a) eine kovalente Bindung,
b) -O-,
c) -CH=CH- oder
d) -C≡C- steht,
B für
a) -(CH₂)ₘ-, worin m die obengenannte Bedeutung hat,
b) -O-(CH₂)_{q}, worin q die ganze Zahl 1, 2, 3, 4 oder 5 bedeutet, oder
c) -CH=CH- steht und
X für -CH=CH-, Sauerstoffatom oder Schwefelatom steht.

Unter dem Begriff "Halogen" wird Fluor, Chlor, Brom oder Jod verstanden. Unter dem Begriff "Alkyl" oder "Alkenyl" werden Kohlenwasserstoffreste verstanden deren Kohlenstoffketten geradkettig oder verzweigt sind. Cyclische Alkylreste sind beispielsweise 3- bis 6-gliedrige Monocyclen wie Cyclopropyl, Cyclobutyl, Cyclopentyl oder Cyclohexyl. Unter dem Begriff "R⁹ der charakteristische Rest einer proteinogenen Aminosäure" werden Reste R verstanden der Formel X_{c}, 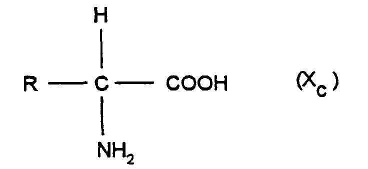 worin R sich von den Aminosäuren Glycin, Alanin, Valin, Leucin, Isoleucin, Phenylalanin, Tyrosin, Tryptophan, Serin, Threonin, Cystein, Methionin, Asparagin, Glutamin, Lysin, Histidin, Arginin, Glutaminsäure und Asparaginsäure ableitet und beide enantiomeren Formen sowie das Racemat oder eine beliebige Mischung eingesetzt werden können. Als geeignete N-Schutzgruppe E werden dafür vorzugsweise die in der Peptidchemie gebräuchlichen N-Schutzgruppen verwendet, beispielsweise Schutzgruppen vom Urethan-Typ, wie Benzyloxycarbonyl(Z), t-Butyloxycarbonyl (Boc), 9-Fluorenylmethoxycarbonyl (Fmoc) und Allyloxycarbonyl (Aloc) oder von Säureamid-Typ, insbesondere Formyl, Acetyl oder Trifluoracetyl von Alkyl-Typ wie Benzyl. Besonders geeignet hat sich dafür auch die (Trimethyl-silyl)ethoxycarbonyl (Teoc)Gruppe bewährt (P. Kociénski, Protecting Groups, Thieme Verlag 1994). Femer können die Alkenylreste auch mehrere Doppelbindungen enthalten.

Die Ausgangsstoffe der chemischen Umsetzungen sind bekannt oder lassen sich nach literaturbekannten Methoden leicht herstellen.

Die Erfindung betrifft ferner ein Verfahren zur Herstellung der Verbindung der Formel I und/oder einer stereoisomeren Form der Verbindung der Formel I und/oder eines physiologisch verträglichen Salzes der Verbindung der Formel I, das dadurch gekennzeichnet ist, daß man
a) die Verbindung der Formel II 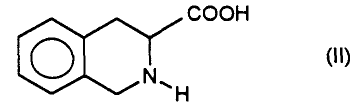
   in eine Verbindung der Formel III überführt, 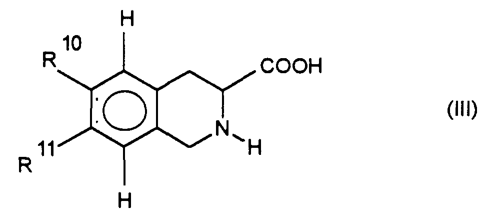 worin R¹⁰ und R¹¹ -NO₂ oder Wasserstoffatom bedeuten und R¹⁰ und R¹¹ nicht gleich sind, und
b) die in a) erhaltene Verbindung der Formel III mit der Verbindung der Formel IV 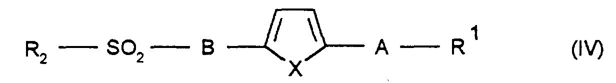 worin B, X, A und R¹ wie in Formel I definiert sind und R₂ Chloratom, Imidazolyl oder -OH bedeutet, in Gegenwart einer Base oder gegebenenfalls eines wasserentziehenden Mittels zu einer Verbindung der Formel V umsetzt. 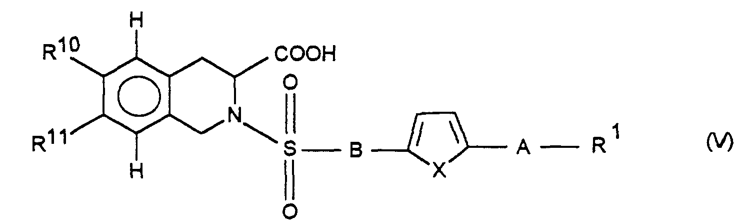 worin R¹⁰ und R¹¹ -NO₂ oder Wasserstoffatom bedeuten und R¹⁰ und R¹¹ nicht gleich sind, und
c) die in b) erhaltene Verbindung der Formel V einer Isomerentrennung unterwirft und eine Verbindung der Formel I erhält, worin R³ und R⁴ zusammen mit dem N-Atom ein NO₂-Rest bilden der in Position 6 oder 7 an dem Phenylrest bindet, oder
d) die in c) erhaltene Verbindung zu einer Verbindung der Formel I reduziert, worin R³ und R⁴ Wasserstoffatom bedeuten, oder
e) eine in d) erhaltene Verbindung mit Carbonsäure- oder Sulfonsäurechloriden, Carbonsäure- oder Sulfonsäureimidazoliden, Chlorameisensäureestern, Aktivestern oder Anhydriden acyliert, oder
f) eine in d) erhaltene Verbindung mit der entsprechenden Aminosäure, Carbonsäure, Aldehyd oder einem gegebenenfalls substituierten Guanidin umsetzt, oder
g) eine in d) erhaltene Verbindung alkyliert, oder
h) eine in a) erhaltene Verbindung zu einer Verbindung der Formel VI umsetzt, 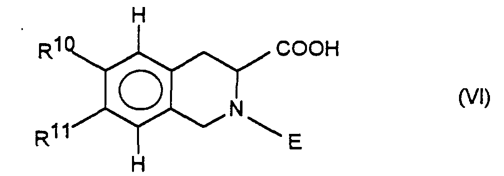 worin E eine N-Schutzgruppe darstellt und R¹⁰ und R¹¹ wie oben definiert sind und
   die Verbindung der Formel VI in die Regioisomere der Formel VII und VIII auftrennt 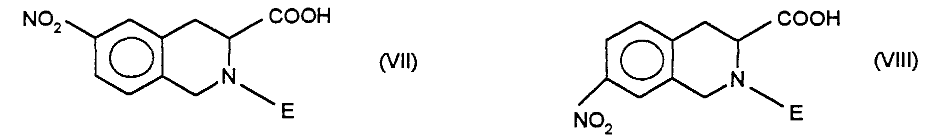 und die Nitrogruppe wie unter d) beschrieben umsetzt und die erhaltene Verbindung wie unter e), f) oder g) umsetzt, oder
i) eine nach den Verfahren h), g), f), e) oder d) erhaltene Verbindung zu den entsprechenden Carbonsäureestem (R² = O-R⁷) umsetzt oder mit Hydroxylamin umsetzt (R² = -N(H)-OH).

Die Erfindung betrifft auch Arzneimittel, gekennzeichnet durch einen wirksamen Gehalt an mindestens einer Verbindung der Formel I und/oder eines physiologisch verträglichen Salzes der Verbindung der Formel I und/oder eine gegebenenfalls stereoisomere Form der Verbindung der Formel I, zusammen mit einem pharmazeutisch geeigneten und physiologisch verträglichen Trägerstoff, Zusatzstoff und/oder anderen Wirk- und Hilfsstoffen.

Aufgrund der pharmakologischen Eigenschaften eignen sich die erfindungsgemäßen Verbindungen zur Prophylaxe und Therapie all solcher Erkrankungen, an deren Verlauf eine verstärkte Aktivität von Matrix-abbauenden Metalloproteinasen beteiligt ist. Dazu gehören degenerative Gelenkerkrankungen wie Osteoarthrosen, Spondylosen, Knorpelschwund nach Gelenktrauma oder längerer Gelenksruhigstellung nach Meniskus- oder Patellaverletzungen oder Bänderrissen. Femer gehören dazu auch Erkrankungen des Bindegewebes wie Kollagenosen, Periodontalerkrankungen, Wundheilungsstörungen und chronische Erkrankungen des Bewegungsapparates wie entzündliche, immunologisch oder stoffwechselbedingte akute und chronische Arthritiden, Arthropathien, Myalgien und Störungen des Knochenstoffwechsels. Ferner eignen sich die Verbindungen der Formel I zur Behandlung der Ulceration, Atherosklerose und Stenosen. Weiterhin eignen sich die Verbindungen der Formel I zur Behandlung von Entzündungen, Krebserkrankungen, Tumormetastasenbildung, Kachexie, Anorexie und septischem Schock.

Die erfindungsgemäßen Arzneimittel werden im allgemeinen oral oder parenteral verabreicht. Die rektale oder transdermale Applikation ist auch möglich.

Die Erfindung betrifft auch ein Verfahren zur Herstellung eines Arzneimittels, das dadurch gekennzeichnet, daß man mindestens eine Verbindung der Formel I mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Geeignete feste oder galenische Zubereitungsformen sind beispielsweise Granulate, Pulver, Dragees, Tabletten (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel, wie Trägerstoffe, Spreng-, Binde-, Überzugs-, Quellungs-, Gleit- oder Schmiermittel, Geschmacksstoffe, Süßungsmittel und Lösungsvermittler, Verwendung finden. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuß- oder Sesamöl, Polyethylenglykol und Lösungsmittel wie etwa steriles Wasser und ein- oder mehrwertige Alkohole wie Glyzerin, genannt.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel I enthält. Bei festen Dosierungseinheiten wie Tabletten, Kapseln, Dragees oder Suppositorien, kann diese Dosis bis zu etwa 1000 mg, bevorzugt jedoch etwa 50 bis 300 mg und bei Injektionslösungen in Ampullenform bis zu etwa 300 mg, vorzugsweise aber etwa 10 bis 100 mg, betragen.

Für die Behandlung eines erwachsenen, etwa 70 kg schweren Patienten sind - je nach Wirksamkeit der Verbindungen gemäß Formel I, Tagesdosen von etwa 20 mg bis 1000 mg Wirkstoff, bevorzugt etwa 100 mg bis 500 mg indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

¹H-NMR-Spektren sind an einem 200-MHz-Gerät der Firma Varian oder einem 400 MHz-Gerät der Firma Bruker aufgenommen worden, in der Regel mit Tetramethylsilan (TMS) als internem Standard und bei Raumtemperatur (RT). Als Lösemittel wurde jeweils DMSO-d₆ verwendet, falls nicht anders vermerkt. Endprodukte werden in der Regel durch massenspektroskopische Methoden (FAB-, ESI-MS) bestimmt. Temperaturangaben in Grad Celsius, RT bedeutet Raumtemperatur (20 °C-26 °C). Verwendete Abkürzungen sind entweder erläutert oder entsprechen den üblichen Konventionen.

### Beispiel 1:

### (6/ 7)-Nitro-1,2,3,4-tetrahydroisochinolin-(R)-3-carbonsäure

100 g 1,2,3,4-Tetrahydroisochinolin-3-carbonsäure (564 mmol) werden bei -10 °C in 500 ml Schwefelsäure (98 %ig, d 1,84) gelöst bzw. suspendiert und auf - 30 °C gekühlt. Anschließend werden 59 g (584 mmol) Kaliumnitrat, gelöst in 200 ml Schwefelsäure und auf 0 °C gekühlt, innerhalb von 1,5 Stunden (h) zugetropft. Die Innentemperatur steigt hierbei wieder auf - 10 °C an. Nach Beendigung der Nitrat-Zugabe wird noch 10 min bei -10 °C und 1 h ohne externe Kühlung gerührt. Man gießt auf Eis neutralisiert unter Kühlung mit konzentrierter wäßriger Ammoniak-Lösung; Verbrauch etwa 1,8 I der 25 %igen Lösung. Vor dem Abfiltrieren der Aminosäure wird mit dem gleichen Volumen Wasser verdünnt. Der erhaltene Feststoff wird erneut in Wasser suspendiert und von verbliebenen löslichen Ammoniumsalzen abfiltriert. Es wird mit viel kaltem Wasser nachgewaschen und unter verminderten Druck bei 60 °C getrocknet.
- Ausbeute:: 110,1 g (88 % der Theorie)
- Schmelzpunkt:: ab 245 °C (langsame Verfärbung), 272-275 °C(schmilzt unter Zersetzung)
- ¹H-NMR:: (400 MHz, DCI/D₂O) 3,05 (dd, 1 H, 7-Isomer); 3,30 (2 dd, überlagert, 2 H, 6- u. 7-lsomer); 3,44 (dd, "1 H", 6-lsomer); 4,25 (m, 3 H); 7,20; 7,80 (2 m, 3 H); Anteil des 6-lsomeren: 13 %
- Elementaranalyse:: C 53,9 (theor. 54,06), H 4,50 (theor. 4,55), N 12,6 (theor. 12,61)
- IR:: 1640 (s), 1540 (s), 1400 (s), 1350 (s) cm-1

### Beispiel 2:

### tert. Butyloxycarbonyl-(6/ 7)-nitro-1,2,3,4-tetrahydroisochinolin-(R)-3-carbonsäure

13,3 g (59,9 mmol) der Verbindung aus Beispiel 1 werden mit 13,1 g (60 mmol) Di-tert-Butyldicarbonat und 12,72 g (120 mmol) Natriumcarbonat in 300 ml Dioxan/Wasser 1:1 gelöst bzw. suspendiert und für 16 h bei Raumtemperatur gerührt. Anschließend wird das Dioxan am Rotationsverdampfer abdestillert und die verbleibende wäßrige Suspension mit 200 ml Essigester überschichtet. Man kühlt auf 5 °C, säuert mit 1 n HCI bis pH 3 an und trennt die organische Phase ab. Diese wird zweimal mit gesättigter NaCI-Lösung gewaschen und über Natriumsulfat getrocknet. Nach dem Abfiltrieren des Trockenmittels wird unter verminderten Druck eingedampft.
- Ausbeute:: 18,1 g (94 % der Theorie)
- Reinheit/Isomerenverteilung:: HPLC-Bestimmung: Nucleosil RP 18,125 x 4 mm, 254 nm, Acetonitril-0,1 m Phosphorsäure 5:95 bis 70:30; 6-Isomer: Retentionszeit 14,19 min., 7-Isomer: Retentionszeit 14,72 min. Verhältnis etwa 1 : 9; Reinheit: 99,0 %
- ¹H-NMR:: (200 MHz) 1,4 (2 s, 9 H); 3,3 (m, 2 H); 4,4-5,0 (3 m, 3 H); 7,4-8,2 (5 m, 3 H);12,7 (s, 1H)

### Beispiel 3:

### 2-tert. Butyloxycarbonyl-7-nitro-1,2,3,4-tetrahydroisochinolin-(R)-3-carbon-säure Dicyclohexylammoniumsalz

Zur Trennung der Regioisomeren werden 10 g der Verbindung aus Beispiel 2 in 300 ml Essigester gelöst und bei Raumtemperatur mit 1 eq. (6,2 ml) Dicyclohexylamin in 10 ml Essigester versetzt.

In der Kälte kristallisiert nach Zugabe von n-Heptan langsam das Dicyclohexylammoniumsalz aus, das nach 16 h abfiltriert und getrocknet wird.

Nach zwei weiteren Umkristallisationen beträgt der Anteil des 6-Isomeren weniger als 1,0 % bei einer Gesamtreinheit größer 99 %. Aus den Mutterlaugen läßt sich weiteres Material gewinnen.
- Ausbeute:: 6,1 g (1. Fraktion)
- Reinheit/Isomerenverteilung:: HPLC-Bestimmung: Nucleosil RP 18, 125 x 4 mm, 254 nm, Acetonitril-0,1 m Phosphorsäure 5:95 bis 70:30; 6-Isomer: Retentionszeit 13,51 min., 7-Isomer: Retentionszeit 14,23 min. Verhältnis > 1 : 99
- ¹H-NMR:: (200 MHz) 0,9-1,9 (mehrere m, ca. 30 H); 2,7-3,05; 3,4; 4,6 (5
- m,: ca. 5 H); 7,4; 8,0 (2 m, 3 H)
- Drehwert:: -23,6° (MeOH, C=1)

### Beispiel 4:

### 2-tert. Butyloxycarbonyl-7-nitro-1,2,3,4-tetrahydroisochinolin-(R)-3-carbonsäure

Zur Freisetzung der geschützten Aminosäure wird das DCHA-Salz aus Beispiel 3 in Essigester gelöst und mit einem Überschuß wäßriger, 10 %iger Citronensäurelösung ausgeschüttelt. Die organische Phase wird mit gesättigter NaCI-Lösung ausgeschüttelt, über Natriumsulfat getrocknet und unter verminderten Druck eingedampft.
- Ausbeute:: zwischen 87 und 95 %
- ¹H-NMR:: Die charakteristischen Signale des Dicyclohexylamins fehlen. Die freigesetzte Verbindung wird unmittelbar weiterverarbeitet.

### Beispiel 5:

### 7-Nitro-1,2,3,4-tetrahydroisochinolin-(R)-3-carbonsäure Hydrochlorid

0,5 g der Verbindung aus Beispiel 4 (1,55 mmol) werden mit 19 ml HCI in Ether versetzt und für 30 min bei RT gerührt, zur Trockne eingedampft, mehrfach mit Toluol coevaporiert und unter verminderten Druck getrocknet.
- Ausbeute:: 0,385 g (96 % der Theorie)
- ¹H-NMR:: (200 MHz) 3,2-3,6 (m, 2 H); 4,3-4,6 (m, 3 H); 7,6 (d, 1 H); 8,1 (dd, 1 H); 8,3 (d, 1 h); 10,5 (s, br., 1 H)
- MS:: 223,1 (M+H)
- Drehwert:: + 143,5° (c=1, MeOH)

### Beispiel 6:

### 2-tert. Butyloxycarbonyl-7-amino-1,2,3,4-tetrahydroisochinolin-(R)-3-carbonsäure

38 g der Nitroverbindung aus Beispiel 4 (117 mmol) werden mit 2 g 10 % Pd auf C in Methanol für 7 h bei RT und leichtem Überdruck an der Parr-Apparatur hydriert. Nach Abdampfen des Lösemittels wird der Rückstand mit Diisopropylether gewaschen und aus Wasser/Ethanol umkristallisiert und abschließend unter verminderten Druck getrocknet.
- Ausbeute:: 33 g (95 % der Theorie)
- ¹H-NMR:: (200 MHz) 1,4 (2 s, 9 H); 2,9 (m, 2 H); 4,2-4,8 (mehrere m, 3 H); 6,4 (m, 2 H); 6,8 (m, 1 H).
- MS:: 293,1 (M+H)
- Drehwert:: + 28,33° (c=1, Methanol)

### Beispiel 7:

### 2-tert. Butyloxycarbonyl-(6/ 7)-amino-1,2,3,4-tetrahydroisochinolin-(R)-3-carbonsäure

Zur Reduktion der Aminosäure aus Beispiel 2 wird wie in Beispiel 6 beschrieben verfahren. Das Rohprodukt wird unter verminderten Druck eingedampft.
- ¹H-NMR:: (200 MHz) 1,4 (2 s, 9 H); 2,9 (m, 2 H); 4,2-4,8 (mehrere m, 3 H); 6,4 (m, breit, 2 H); 6,8 (m, 1 H).
- MS:: 293,1 (M+H)

### Beispiel 8:

### 2-tert. Butyloxycarbonyl-7-amino-1,2,3,4-tetrahydroisochinolin-(R)-3-carbonsäure (Alternativverfahren)

Die Isomerenmischung aus Beispiel 7 wird in der Siedehitze mit Acetonitril behandelt. Nach dem Erkalten wird abfiltriert. Diese Behandlung wird 2-3 mal durchgeführt.
- ¹H-NMR:: (200 MHz) 1,4 (2 s, 9 H); 2,9 (m, 2 H); 4,2-4,8 (mehrere m, 3 H); 6,4 (m, 2 H); 6,8 (m, 1 H); kein Unterschied zu Beispiel 6.
- MS:: 293,1 (M+H)
- Drehwert:: + 28,13° (c=1, Methanol)

### Beispiel 9:

### 7-Amino-1,2,3,4-tetrahydroisochinolin-(R)-3-carbonsäure Dihydrochlorid

0,5 g (1,7 mmol) der Verbindung aus Beispiel 8 werden bei RT für 30 min mit HCI in Ether behandelt. Nach Eindampfen unter verminderten Druck wird mit Toluol coevaporiert und das Produkt wird im Ölpumpenvakuum von Lösemittelresten befreit.
- Ausbeute:: 0,41 g (91 % der Theorie)
- ¹H-NMR:: (200 MHz) 3,0-3,5 (m, 2 H); 4,2-4,5 (m, 3 H); 7,1-7,4 (2 m, 3H); 10,0 (s, breit, 1 H)
- MS:: 193,0 (M + H)
- Drehwert:: + 86,3° (C=1, Methanol)

### Beispiel 10:

### 2-(4-Methoxybenzolsulfonyl)-7-amino-1,2,3,4-tetrahydroisochinolin-(R)-3-( N-hydroxy)-carboxamid

2-(4-Methoxybenzolsulfonyl)-7-(tert. butoxycarbonyl)-amino-1,2,3,4-tetrahydroisochinolin-(R)-3-carbonsäure, wird unter Standardbedingungen, die dem Fachmann bekannt sind, aus der in Beispiel 1 angeführten Verbindung auf dem in der Verfahrensvariante a) angeführten Weg (Sulfonamidbildung mit 4-Methoxybenzolsulfonsäurechlorid, chromatographische Aufreinigung der 6-/7-Isomeren (Beispiel 13), Reduktion der Nitrogruppe zur Aminogruppe (Beispiel 12) und Einführung der Boc-Schutzgruppe) erhalten.

Zur Darstellung der Hydroxamsäure werden 10 g (22 mmol) 2-(4-Methoxybenzolsulfonyl)-7-(tert. butoxycarbonyl)-amino-1,2,3,4-tetrahydroisochinolin-(R)-3-carbonsäure in 100 ml Tetrahydrofuran (THF) gelöst, auf - 15 °C gekühlt und nacheinander mit 2,1 ml (22 mmol) Chlorameisensäureethylester, 4,8 ml (44 mmol) N-Methylmorpholin und nach 45 min bei dieser Temperatur mit 13,5 ml (110 mmol) O-Trimethylsilylhydroxylamin versetzt. Man rührt noch 3 h bei RT, entfernt das Lösemittel unter verminderten Druck, nimmt in Essigester auf und schüttelt nacheinander mit 10 % iger Citronensäurelösung, 10 % iger Natriumcarbonatlösung und gesättigter NaCl-Lösung aus, trocknet über Natriumsulfat, dampft am Rotationsverdampfer ein und entfernt Lösemittelreste im Ölpumpenvakuum.

2,6 g dieser als Beispiel 11 angeführten Verbindung (Gesamtausbeute 9,1 g) werden nach chromatographischer Aufreinigung mit 50 ml HCI in Diethylether versetzt und für 30 min bei RT gerührt. Anschließend wird unter verminderten Druck eingedampft und der Rückstand mit Toluol coevaporiert.
- Ausbeute:: 1,97 g (89 % der Theorie)
- ¹H-NMR:: 2,75 (m, 2 H); 3,8 (s, 3 H); 4,40 (m, 3 H); 6,9-7,3 (m, 3 H); 7,0: 7,7 (2 d, 4 H); 8,8; 9,3; 10,7 (3 s, 3 H)

Die in der folgenden Tabelle 1 genannten Verbindungen sind analog zu den vorhergehenden Beispielen hergestellt worden.

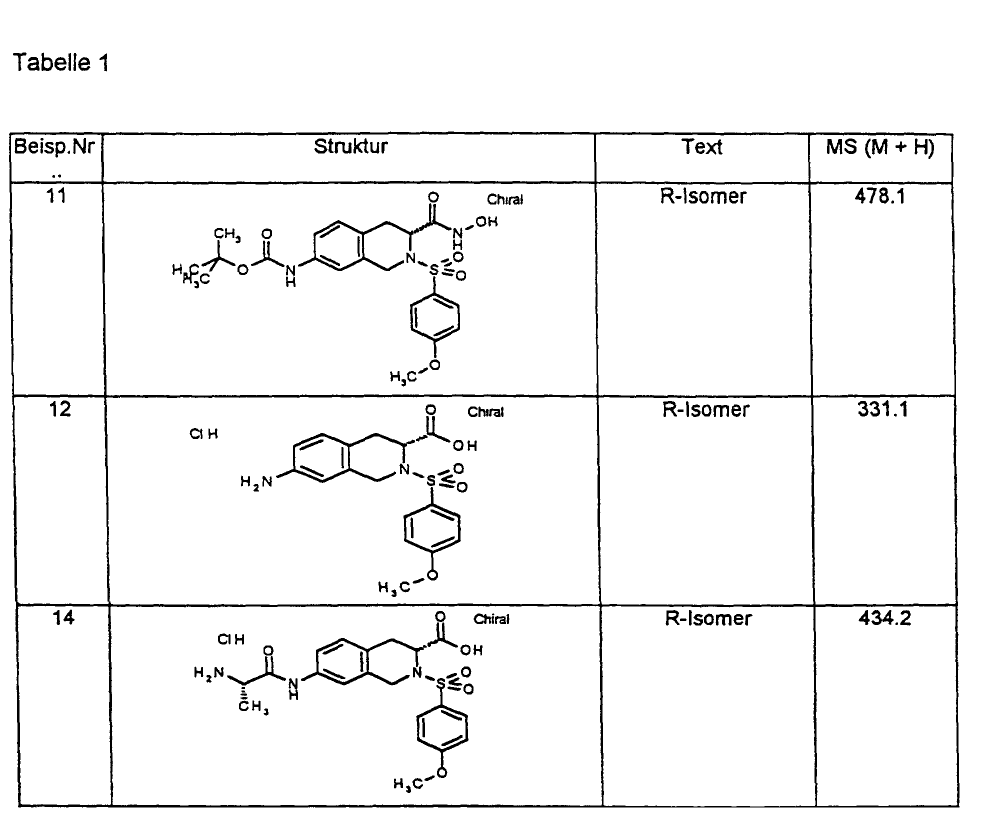

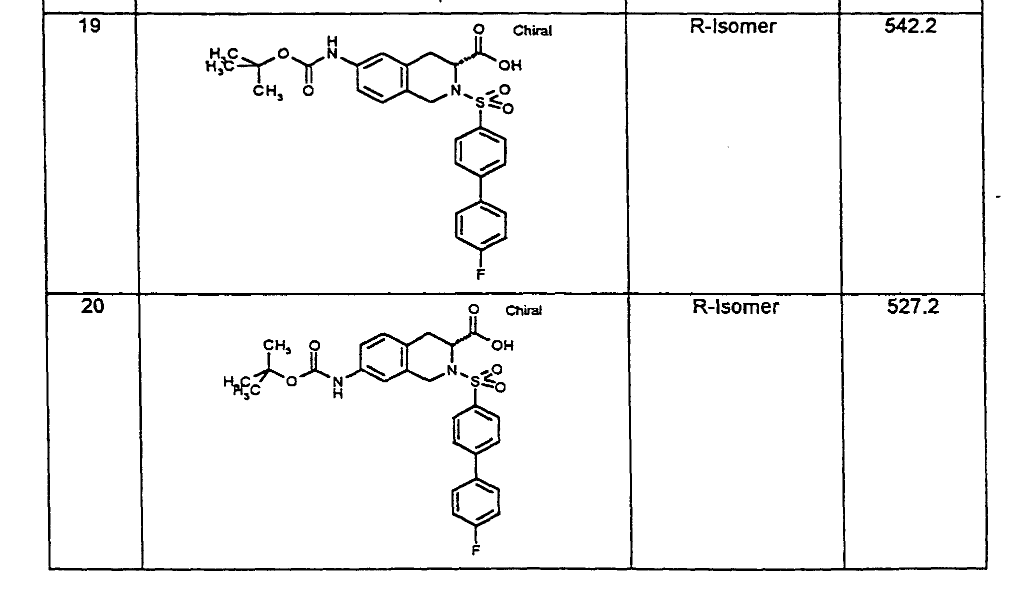

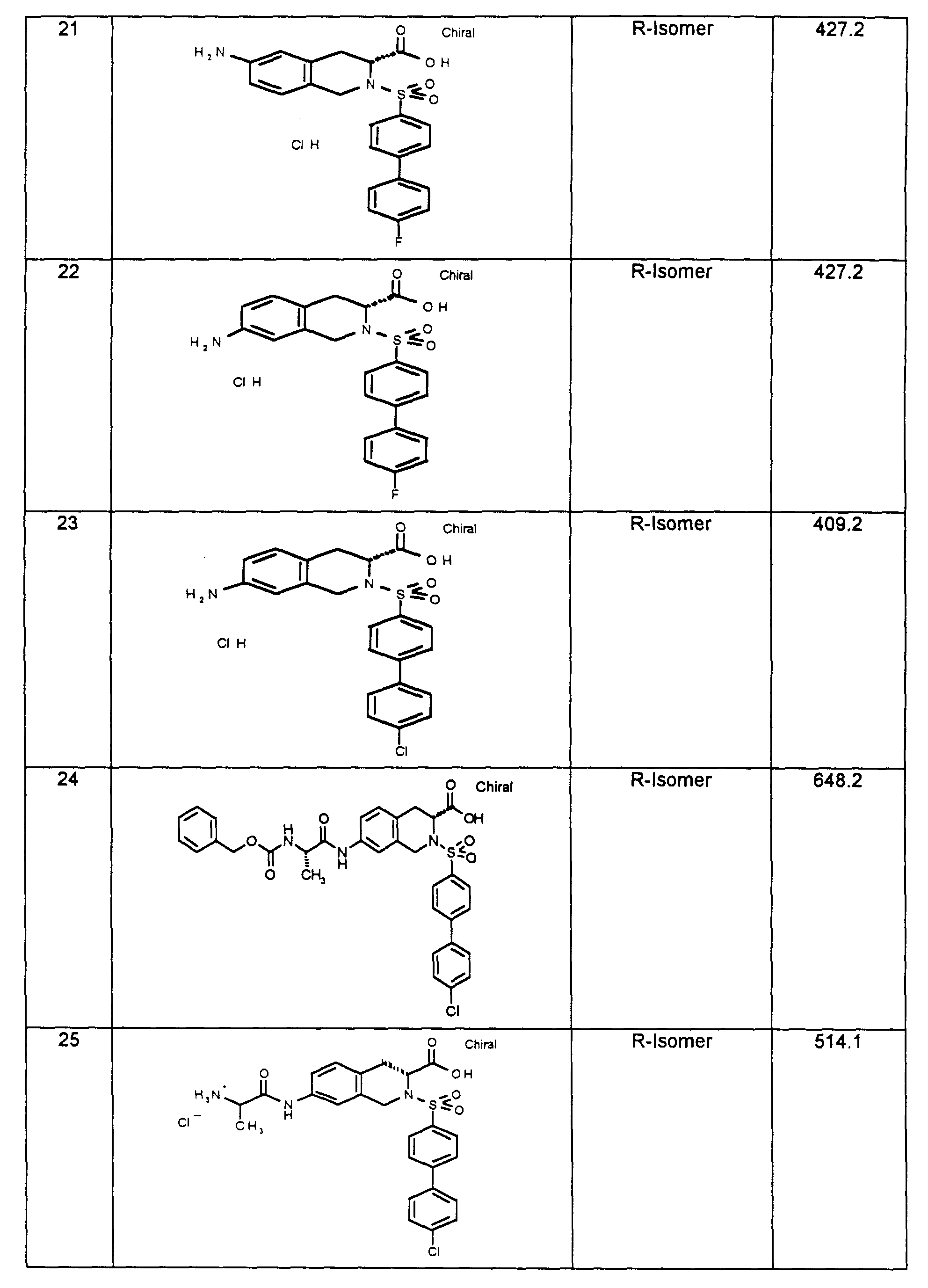

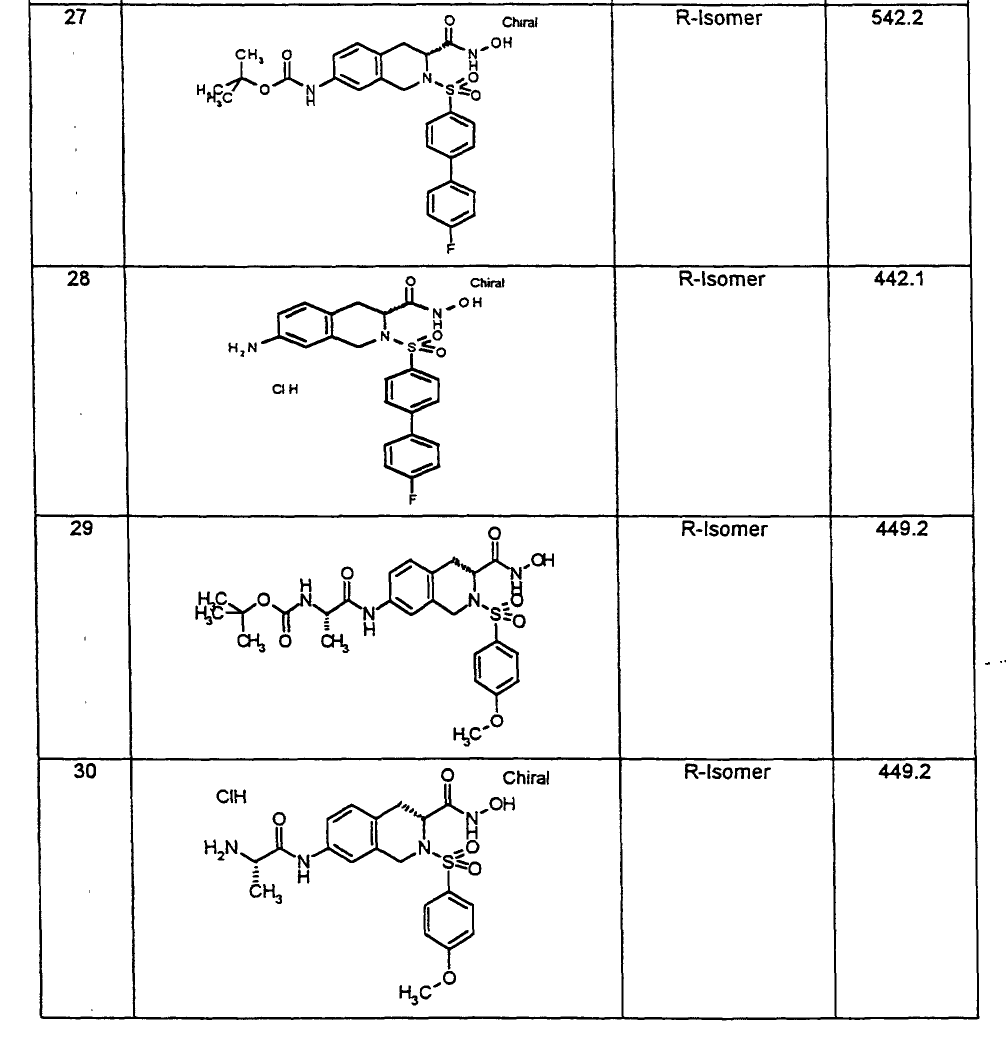

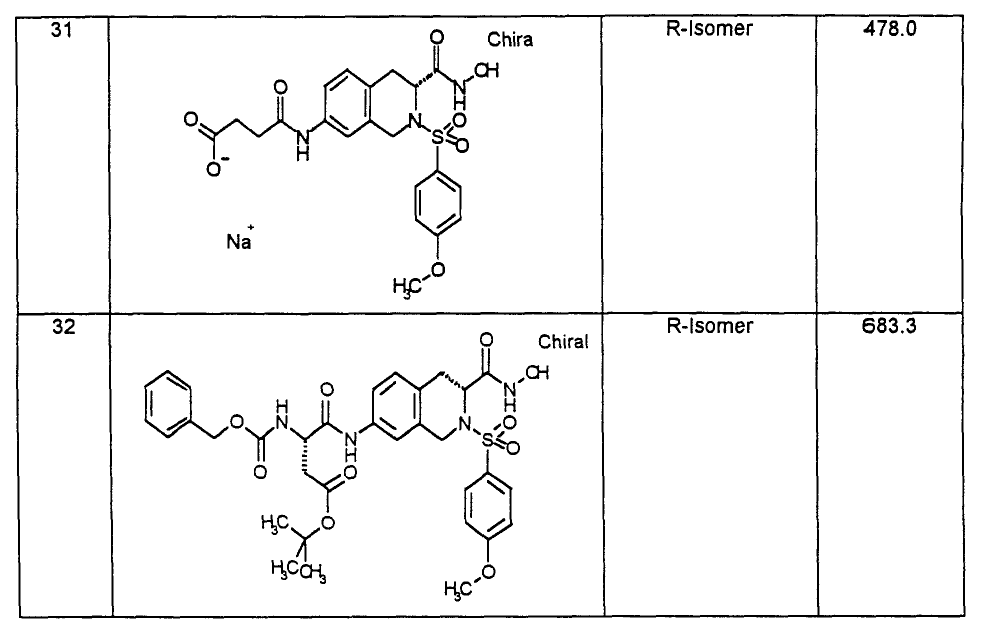

### Pharmakologische Beispiele

### Darstellung und Bestimmung der enzymatischen Aktivität der katalytischen Domäne des humanen Stromelysins und der Neutrophilen-Kollagenase.

Die beiden Enzyme -Stromelysin (MMP-3) und Neutrophilen-Kollagenase (MMP-8)-wurden dargestellt nach Ye et al. (Biochemistry; 31 (1992) Seiten 11231-11235). Zur Messung der Enzymaktivität oder der Enzyminhibitorwirkung werden 70 µl Pufferlösung, und 10 µl Enzymlösung mit 10 µl einer 10%igen (v/v) wäßrigen Dimethylsulfoxid-Lösung, die gegebenenfalls den Enzyminhibitor enthält, für 15 Minuten inkubiert. Nach Zugabe von 10 µl einer 10%igen (v/v) wäßrigen Dimethylsulfoxid-Lösung, die 1 mmol/l des Substrates enthält, wird die Enzymreaktion fluoreszenzspektroskopisch verfolgt (328 nm (ex) /393 nm(em)).

Die Enzymaktivität wird dargestellt als Extinktionszunahme/Minute. Die in Tabelle 2 aufgeführten IC₅₀-Werte werden als diejenige Inhibitorkonzentrationen ermittelt, die jeweils zu einer 50%igen Inhibierung des Enzyms führen.

Die Pufferlösung enthält 0,05% Brij (Sigma, Deisenhofen, Deutschland) sowie 0,1 mol/l Tris/HCl, 0,1 mol/l NaCI, 0,01 mol/l CaCl₂ und 0,1 mol/l Piperazin-N,N'-bis[2-ethansulfonsäure] (pH=6,5).

Die Enzymlösung enthält 5 µg/ml einer der nach Ye et al. dargestellten Enzymdomänen. Die Substratlösung enthält 1 mmol/l des fluorogenen Substrates (7-Methoxycoumarin-4-yl)acetyl-Pro-Leu-Gly-Leu-3-(2',4'-dinitrophenyl)-L-2,3-diaminopropionyl-Ala-Arg-NH₂ (Bachem, Heidelberg, Deutschland).

**Tabelle 2**

| **Beisp. Nr.** | **MMP-3** | **MMP-8** |
|---|---|---|
| | | |
| 10 | 1 10⁻⁸ | 2 10⁻⁹ |
| 11 | 2 10⁻⁸ | 3 10⁻⁹ |
| 15 | 6 10⁻⁷ | 3 10⁻⁸ |
| 16 | 5 10⁻⁷ | 2 10⁻⁸ |
| 17 | 1 10⁻⁶ | 4 10⁻⁸ |
| 18 | 5 10⁻⁷ | 2 10⁻⁸ |
| 19 | 4 10⁻⁷ | 3 10⁻⁸ |
| 20 | 2 10⁻⁶ | 1 10⁻⁷ |
| 21 | 2 10⁻⁷ | 8 10⁻⁹ |
| 22 | 3 10⁻⁷ | 8 10⁻⁹ |
| 23 | 2 10⁻⁷ | 7 10⁻⁹ |
| 24 | 3 10⁻⁷ | 6 10⁻⁸ |
| 25 | 2 10⁻⁷ | 1 10⁻⁸ |
| 26 | 8 10⁻⁸ | 8 10⁻⁹ |
| 27 | 1 10⁻⁷ | 1 10⁻⁸ |
| 28 | 2 10⁻⁸ | 2 10⁻⁹ |
| 29 | 2 10⁻⁸ | 2 10⁻⁹ |
| 30 | 3 10⁻⁸ | 5 10⁻⁹ |
| 31 | 2 10⁻⁸ | 4 10⁻⁹ |
| 32 | 4 10⁻⁷ | 1 10⁻⁸ |

## Patentansprüche

1. Verbindung der Formel I 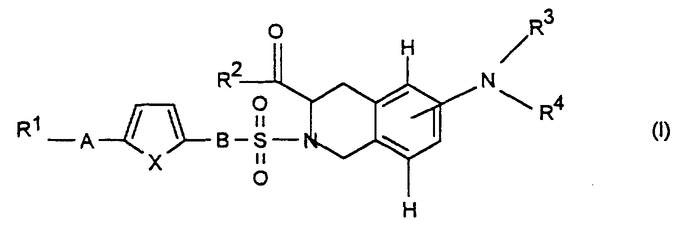 und/oder eine stereoisomere Form der Verbindung der Formel I und/oder ein physiologisch verträgliches Salz der Verbindung der Formel I, wobei
R¹ für
1. Phenyl,
2. Phenyl, welches ein- oder zweifach substituiert ist durch
2.1. (C₁-C₆)-Alkyl, gerade, cyclisch oder verzweigt,
2.2. -OH,
2.3. (C₁-C₆)-Alkyl-C(O)-O-,
2.4. (C₁-C₆)-Alkyl-O-,
2.5. (C₁-C₆)-Alkyl-O-(C₁-C₄)-Alkyl-O-,
2.6. Halogen,
2.7. -CF₃,
2.8. -CN,
2.9. -NO₂,
2.10. HO-C(O)-,
2.11. (C₁-C₆)-Alkyl-O-C(O)-,
2.12. Methylendioxo,
2.13. R⁵-(R⁶ )N-C(O)-, worin R⁵ und R⁶ gleich oder verschieden sind und für Wasserstoffatom oder (C₁-C₆)-Alkyl- stehen, oder
2.14. R⁵-(R⁶ )N-, worin R⁵ und R⁶ gleich oder verschieden sind und für Wasserstoffatom oder (C₁-C₆)-Alkyl- stehen,
3. einen Heteroaromaten aus der nachfolgenden Gruppe 3.1. bis 3.15., der unsubstituiert oder wie unter 2.1 bis 2.14 beschrieben substituiert ist,
3.1. Pyrrol,
3.2. Pyrazol,
3.3. Imidazol,
3.4. Triazol,
3.5. Thiophen,
3.6. Thiazol,
3.7. Oxazol,
3.8. Isoxazol,
3.9. Pyridin,
3.10. Pyrimidin,
3.11. Indol,
3.12 Benzothiophen,
3.13. Benzimidazol,
3.14. Benzoxazol oder
3.15. Benzothiazol,
4. -OH steht und A eine kovalente Bindung bedeutet,
5. -O-R¹⁴ steht und A eine kovalente Bindung, -CH=CH- oder -C≡C- bedeutet und worin R¹⁴
1) (C₁-C₆)-Alkyl,
2) (C₃-C₆)-Cycloalkyl,
3) Benzyl oder
4) Phenyl bedeutet,
6. -COOH steht und A eine kovalente Bindung, -CH=CH- oder -C≡C- bedeutet,
7. (C₁-C₆)-Alkyl,
8. (C₃-C₆)-Cycloalkyl-O-(C₁-C₄)-alkyl,
9. Halogen steht und A eine kovalente Bindung, -CH=CH- oder -C≡C- bedeutet,
10. -CN steht und A eine kovalente Bindung, -CH=CH- oder -C≡C- bedeutet,
11. -NO₂ steht und A eine kovalente Bindung, -CH=CH- oder -C≡C- bedeutet, oder
12. -CF₃ steht, und
R² für
1. HO(H)N- oder
2. R⁷-O- steht, worin R⁷
2.1 Wasserstoffatom,
2.2 (C₁-C₆)-Alkyl,
2.3 Allyl oder
2.4 Benzyl bedeutet,
R³ und R⁴ gleich oder verschieden sind und für
1. Wasserstoffatom,
2. (C₁-C₆)-Alkyl,
3. Phenyl-(CH₂)ₘ, worin Phenyl unsubstituiert oder ein- oder zweifach wie unter 2.1 bis 2.14. beschrieben substituiert ist und m die ganze Zahl Null, 1, 2 oder 3 bedeutet,
4. R⁸-(CO)-, worin R⁸
4.1 (C₁-C₈)-Alkyl,
4.2 Phenyl-(CH₂)ₘ-, worin Phenyl unsubstituiert oder einoder zweifach wie unter 2.1. bis 2.14. beschrieben substituiert ist und m die ganze Zahl Null, 1, 2 oder 3 darstellt,
4.3 R⁷-O-C(O)-(CH₂)ₙ-, worin R⁷ wie oben definiert ist und n die ganze Zahl Null, 1, 2, 3, 4, 5 oder 6 bedeutet,
4.4 R⁷-N(H)-(R⁹)-C(H)-, worin R⁷ wie oben definiert ist und R⁹ den charakteristischen Rest einer proteinogenen α-Aminosäure bedeutet und worin R⁹ unsubstituiert oder an einem Sauerstoff- oder Schwefelatom ein- oder zweifach durch (C₁-C₄)-Alkyl, Benzyl, Allyl substituiert ist oder durch eine N-Schutzgruppe substituiert ist,
4.5 R⁷-C(O)-N(H)-(R⁹)-C(H)-, worin R⁷ und R⁹ wie unter 4.4 definiert sind, oder
4.6 R¹⁰-O-C(O)-N(H)-(R⁹)-C(H)-, R⁹ wie unter 4.4 definiert ist und worin R¹⁰
4.6.1 (C₁-C₆)-Alkyl,
4.6.2 Allyl,
4.6.3 Benzyl oder
4.6.4 (9-Fluorenyl)-methyl bedeutet,
5. R¹⁰-O-C(O)-, worin R¹⁰ wie unter 4.6.1 bis 4.6.4 definiert ist,
6. R¹⁵-SO₂-, worin R¹⁵
6.1 (C₁-C₆)-Alkyl,
6.2 Allyl oder
6.3 Phenyl-(CH₂)ₘ-, worin Phenyl unsubstituiert oder ein- oder zweifach wie unter 2.1 bis 2.14 beschrieben substituiert ist und m die ganze Zahl Null, 1, 2 oder 3 darstellt, oder
7. H₂N-C(=NH)- stehen, oder
R³ und R⁴ zusammen mit dem N-Atom einen Rest der Formel Xₐ oder X_{b} bilden, 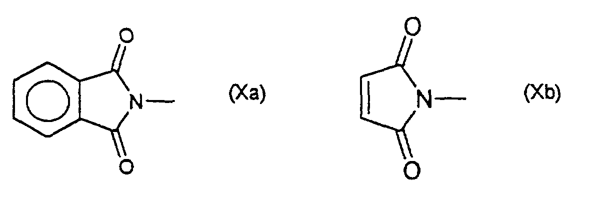 A für
a) eine kovalente Bindung,
b) -O-,
c) -CH=CH- oder
d) -C≡C- steht,
B für
a) -(CH₂)ₘ-, worin m die obengenannte Bedeutung hat,
b) -O-(CH₂)_{q}, worin q die ganze Zahl 1, 2, 3, 4 oder 5 bedeutet, oder
c) -CH=CH- steht und
X für -CH=CH-, Sauerstoffatom oder Schwefelatom steht.

2. Verfahren zur Herstellung der Verbindung der Formel I gemäß Anspruch 1, **dadurch gekennzeichnet, daß** man
a) die Verbindung der Formel II 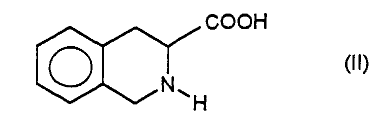 in eine Verbindung der Formel III überführt, 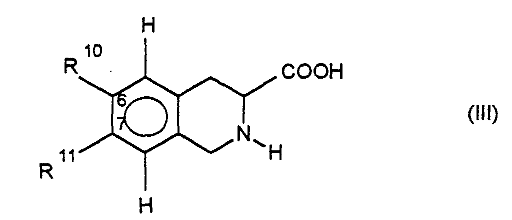 worin R¹⁰ und R¹¹ -NO₂ oder Wasserstoffatom bedeuten und R¹⁰ und R¹¹ nicht gleich sind, und
b) die in a) erhaltene Verbindung der Formel III mit der Verbindung der Formel IV 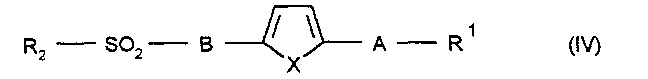 worin B, X, A und R¹ wie in Formel I definiert sind und R₂ Chloratom, Imidazolyl oder -OH bedeutet, in Gegenwart einer Base oder gegebenenfalls eines wasserentziehenden Mittels zu einer Verbindung der Formel V umsetzt, 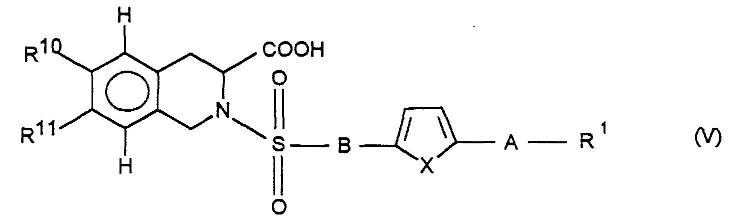 worin R¹⁰ und R¹¹ -NO₂ oder Wasserstoffatom bedeuten und R¹⁰ und R¹¹ nicht gleich sind, und
c) die in b) erhaltene Verbindung der Formel V einer Isomerentrennung unterwirft und eine Verbindung der Formel I erhält, worin R³ und R⁴ zusammen mit dem N-Atom ein NO₂-Rest bilden der in Position 6 oder 7 an dem Phenylrest bindet, oder
d) die in c) erhaltene Verbindung zu einer Verbindung der Formel I reduziert, worin R³ und R⁴ Wasserstoffatom bedeuten, oder
e) eine in d) erhaltene Verbindung mit Carbonsäure- oder Sulfonsäurechloriden, Carbonsäure- oder Sulfonsäureimidazoliden, Chlorameisensäureestern, Aktivestern oder Anhydriden acyliert, oder
f) eine in d) erhaltene Verbindung mit der entsprechenden Aminosäure, Carbonsäure, Aldehyd oder einem gegebenenfalls substituierten Guanidin umsetzt, oder
g) eine in d) erhaltene Verbindung alkyliert, oder
h) eine in a) erhaltene Verbindung zu einer Verbindung der Formel VI umsetzt, 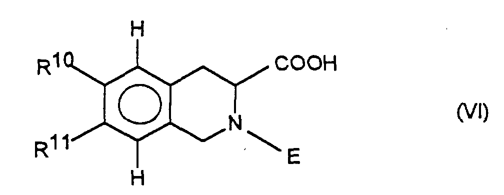 worin E eine N-Schutzgruppe darstellt und R¹⁰ und R¹¹ wie oben definiert sind und die Verbindung der Formel VI in die Regioisomere der Formel VII und VIII auftrennt 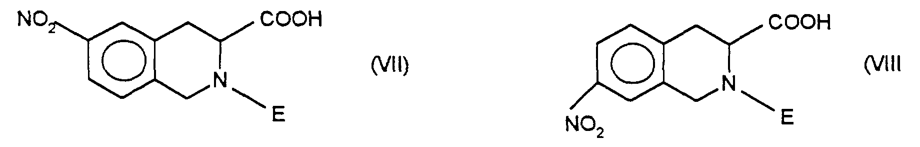 und die Nitrogruppe wie unter d) beschrieben umsetzt und die erhaltene Verbindung wie unter e), f) oder g) umsetzt,
i) eine nach den Verfahren h), g), f), e) oder d) erhaltene Verbindung zu den entsprechenden Carbonsäureestem (R² = O-R⁷) umsetzt oder mit Hydroxylamin umsetzt (R² = -N(H)-OH).

3. Arzneimittel, **gekennzeichnet durch** einen wirksamen Gehalt an mindestens einer Verbindung der Formel I gemäß Anspruch 1 zusammen mit einem pharmazeutisch geeigneten und physiologisch verträglichen Trägerstoff, Zusatzstoff und/oder anderen Wirk- und Hilfsstoffen.

4. Verwendung von mindestens einer Verbindung der Formel I gemäß Anspruch 1 zur Herstellung von Arzneimitteln zur Prophylaxe und Therapie von Erkrankungen, an deren Verlauf eine verstärkte Aktivität von Matrix-abbauenden Metalloproteinasen beteiligt ist.

5. Verwendung gemäß Anspruch 4 für die Behandlung von degenerativen Gelenkerkrankungen wie Osteoarthrosen, Spondylosen, Knorpelschwund nach Gelenktrauma oder längerer Gelenksruhigstellung nach Meniskus- oder Patellaverletzungen oder Bänderrissen, Erkrankungen des Bindegewebes wie Kollagenosen, Periodontalerkrankungen, Wundheilungsstörungen und chronische Erkrankungen des Bewegungsapparates wie entzündliche, immunologisch oder stoffwechselbedingte akute und chronische Arthritiden, Arthropathien, Myalgien und Störungen des Knochenstoffwechsels, der Ulceration, Atherosklerose und Stenosen, aber auch zur Behandlung von Entzündungen, Krebserkrankungen, Tumormetastasenbildung, Kachexie, Anorexie und septischem Schock.

6. Verfahren zur Herstellung eines Arzneimittels , **dadurch gekennzeichnet, daß** man mindestens eine Verbindung der Formel I gemäß Anspruch 1 mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

## Claims

1. A compound of the formula I 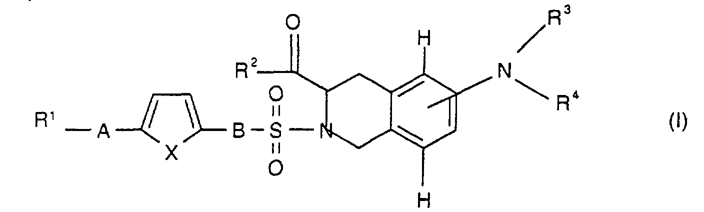 and/or a stereoisomeric form of the compound of the formula I and/or a physiologically tolerable salt of the compound of the formula I, where
R¹ is
1. phenyl,
2. phenyl which is mono- or disubstituted by
2.1. (C₁-C₆)-alkyl, which is linear, cyclic or branched,
2.2. -OH,
2.3. (C₁-C₆)-alkyl-C(O)-O-,
2.4. (C₁-C₆)-alkyl-O-,
2.5. (C₁-C₆)-alkyl-O-(C₁-C₄)-alkyl-O-,
2.6. halogen,
2.7. -CF₃,
2.8. -CN,
2.9. -NO₂,
2.10. HO-C(O)-,
2.11. (C₁-C₆)-alkyl-O-C(O)-,
2.12. methylenedioxo,
2.13. R⁵-(R⁶)N-C(O)-, in which R⁵ and R⁶ are identical or different and represent a hydrogen atom or (C₁-C₆)-alkyl-,
or
2.14. R⁵-(R⁶)N-, in which R⁵ and R⁶ are identical or different and represent a hydrogen atom or (C₁-C₆)-alkyl-,
3. a heteroaromatic from the following group 3.1. to 3.15., which is unsubstituted or substituted as described under 2.1 to 2.14,
3.1. pyrrole,
3.2. pyrazole,
3.3. imidazole,
3.4. triazole,
3.5. thiophene,
3.6. thiazole,
3.7. oxazole,
3.8. isoxazole,
3.9. pyridine,
3.10. pyrimidine,
3.11. indole,
3.12 benzothiophene,
3.13. benzimidazole,
3.14. benzoxazole or
3.15. benzothiazole,
4. -OH and A is a covalent bond,
5. -O-R¹⁴ and A is a covalent bond, -CH=CH- or -C≡C- and in which R¹⁴ is
1) (C₁-C₆)-alkyl,
2) (C₃-C₆)-cycloalkyl,
3) benzyl or
4) phenyl,
6. -COOH and A is a covalent bond, -CH=CH- or -C≡C-,
7. (C₁-C₆)-alkyl,
8. (C₃-C₆)-cycloalkyl-O-(C₁-C₄)-alkyl,
9. halogen and A is a covalent bond, -CH=CH- or -C≡C-,
10. -CN and A is a covalent bond, -CH=CH- or -C≡C-,
11. -NO₂ and A is a covalent bond, -CH=CH- or -C≡C-, or
12. -CF₃, and
R² is
1. HO(H)N- or
2. R⁷-O-, in which R⁷ is
2.1 a hydrogen atom,
2.2 (C₁-C₆)-alkyl,
2.3 allyl or
2.4 benzyl,
R³ and R⁴ are identical or different and are
1. a hydrogen atom,
2. (C₁-C₆)-alkyl,
3. phenyl-(CH₂)ₘ, in which phenyl is unsubstituted or mono- or disubstituted as described under 2.1 to 2.14. and m is the integer zero, 1, 2 or 3,
4. R⁸-(CO)-, in which R⁸ is
4.1 (C₁-C₈)-alkyl,
4.2 phenyl-(CH₂)ₘ-, in which phenyl is unsubstituated or mono- or disubstituted as described under 2.1. to 2.14. and m is the integer zero, 1, 2 or 3,
4.3 R⁷-O-C(O)-(CH₂)ₙ-, in which R⁷ is as defined above and n is the integer zero, 1, 2, 3, 4, 5 or 6,
4.4 R⁷-N(H)-(R⁹)-C(H)-, in which R⁷ is as defined above and R⁹ is the characteristic radical of a proteinogenic α-amino acid and in which R⁹ is unsubstituted or mono- or disubstituted on an oxygen or sulfur atom by (C₁-C₄)-alkyl, benzyl or allyl or is substituted by an N-protective group,
4.5 R⁷-C(O)-N(H)-(R⁹)-C(H)-, in which R⁷ and R⁹ are as defined under 4.4, or
4.6 R¹⁰-O-C(O)-N(H)-(R⁹)-C(H)-, in which R⁹ is as defined under
4.4 and in which R¹⁰ is
4.6.1 (C₁-C₆)-alkyl,
4.6.2 allyl,
4.6.3 benzyl or
4.6.4 (9-fluorenyl)methyl,
5. R¹⁰-O-C(O)-, in which R¹⁰ is as defined under 4.6.1 to 4.6.4,
6. R¹⁵-SO₂-, in which R¹⁵ is
6.1 (C₁-C₆)-alkyl,
6.2 allyl or
6.3 phenyl-(CH₂)ₘ-, in which phenyl is unsubstituted or mono- or disubstituted as described under 2.1 to 2.14 and m is the integer zero, 1, 2 or 3, or
7. H₂N-C(=NH)-, or
R³ and R⁴ together with the nitrogen atom form a radical of the formula Xₐ or X_{b}, 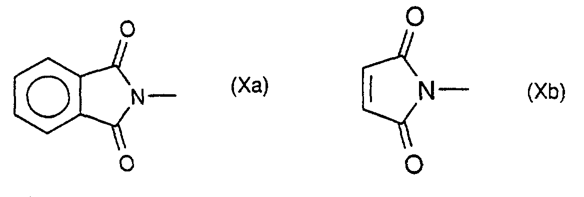 A is
a) a covalent bond,
b) -O-,
c) -CH=CH- or
d) -C≡C-,
B is
a) -(CH₂)ₘ-, in which m has the abovementioned meaning,
b) -O-(CH₂)_{q}, in which q is the integer 1, 2, 3, 4 or 5, or
c) -CH=CH- and
X is -CH=CH-, an oxygen atom or sulfur atom.

2. A process for the preparation of the compound of the formula I as claimed in claim 1, which comprises
a) converting the compound of the formula II 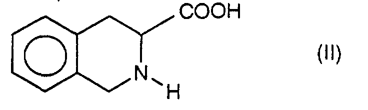 into a compound of the formula III, 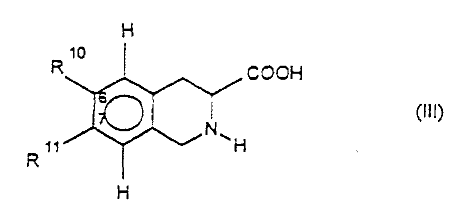 in which R¹⁰ and R¹¹ are -NO₂ or a hydrogen atom and R¹⁰ and R¹¹ are not identical, and
b) reacting the compound of the formula III obtained in a) with the compound of the formula IV 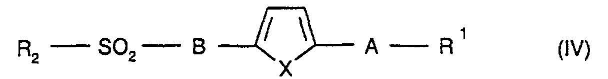 in which B, X, A and R¹ are as defined in formula I and R₂ is a chlorine atom, imidazolyl or -OH, in the presence of a base or, if appropriate, a dehydrating agent to give a compound of the formula V 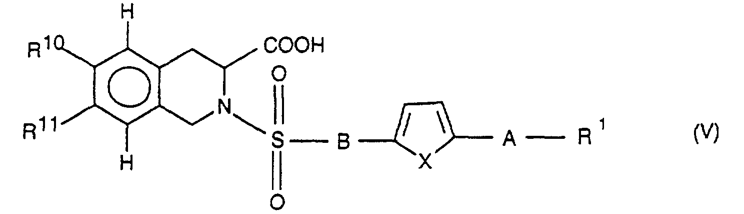 in which R¹⁰ and R¹¹ are -NO₂ or a hydrogen atom and R¹⁰ and R¹¹ are not identical, and
c) subjecting the compound of the formula V obtained in b) to an isomer separation and obtaining a compound of the formula I in which R³ and R⁴ together with the nitrogen atom form an NO₂ radical which binds to the phenyl radical in position 6 or 7, or
d) reducing the compound obtained in c) to a compound of the formula I in which R³ and R⁴ are hydrogen, or
e) acylating a compound obtained in d) with carbonyl or sulfonyl chlorides, carboxylic or sulfonic imidazolides, chloroformic acid esters, active esters or anhydrides, or
f) reacting a compound obtained in d) with the appropriate amino acid, carboxylic acid, aldehyde or an optionally substituted guanidine, or
g) alkylating a compound obtained in d), or
h) reacting a compound obtained in a) to give a compound of the formula VI, 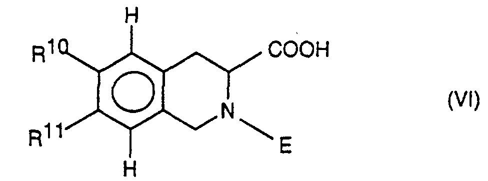 in which E is a N-protective group and R¹⁰ and R¹¹ are as defined above and separating the compound of the formula VI into the regioisomers of the formulae VII and VIII 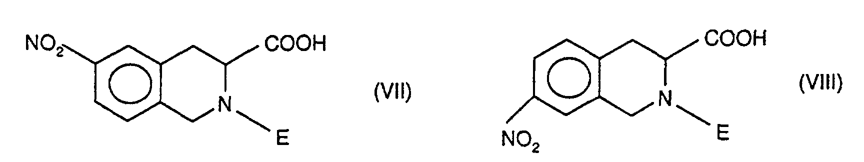 and reacting the nitro group as described under d) and reacting the compound obtained as under e), f) or g), or
i) reacting a compound obtained by the process h), g), f), e) or d) to give the corresponding carboxylic acid esters (R² = O-R⁷) or reacting it with hydroxylamine (R² = -N(H)-OH).

3. A pharmaceutical, comprising an efficaceous amount of at least one compound of the formula I as claimed in claim 1 together with a pharmaceutically suitable and physiologically tolerable excipient, additive and/or other active compounds and auxiliaries.

4. The use of at least one compound of the formula I as claimed in claim 1 for the production of pharmaceuticals for the prophylaxis and therapy of disorders in the course of which an increased activity of matrix-degrading metalloproteinases is involved.

5. The use as claimed in claim 4 for the treatment of degenerative joint disorders such as osteoarthroses, spondyloses, chondrolysis after joint trauma or relatively long joint immobilization after meniscus or patella injuries or ligament tears, disorders of the connective tissue such as collagenoses, periodontal disorders, wound healing disorders and chronic disorders of the locomotory apparatus such as inflammatory, immunologically or metabolically related acute and chronic arthritides, arthropathies, myalgias and disorders of the bone metabolism, ulceration, atherosclerosis and stenoses, but also for the treatment of inflammations, carcinomatous disorders, formation of tumor metastases, cachexia, anorexia and septic shock.

6. Process for the production of a pharmaceutical, **characterized in that** at least one compound of the formula I as claimed in claim 1 is brought into a suitable administration form using a pharmaceutically suitable and physiologically tolerable excipient and, if appropriate, further suitable active compounds, additives or auxiliaries.

## Revendications

1. Composé de formule I 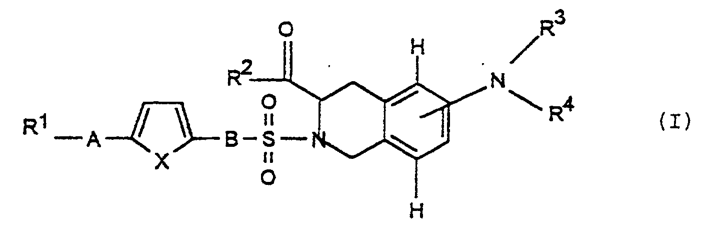 et/ou une forme stéréoisomère du composé de formule I et/ou un sel du composé de formule I toléré sur le plan physiologique, où
R¹ représente un groupe
1. phényle,
2. phényle qui est substitué une ou deux fois par des substituants
2.1. alkyle un C₁-C₆, linéaire, cyclique ou ramifié,
2.2. -OH,
2.3. (alkyle en C₁-C₆)-C(O)-O-,
2.4. (alkyle en C₁-C₆)-O-,
2.5. (alkyl en C₁-C₆)-O-(alkyl en C₁-C₄)-O-,
2.6. halogène,
2.7. -CF₃,
2.8. -CN,
2.9. -NO₂,
2.10. HO-C(O)-,
2.11. (alkyl en C₁-C₆)-O-C(O)-,
2.12. méthylènedioxo,
2.13. R⁵-(R⁶)NC(O)-, où R⁵ et R⁶ sont identiques ou différents et représentent des atomes d'hydrogène ou des groupes alkyle en C₁-C₆,
2.14. R⁵-(R⁶)N-, où R⁵ et R⁶ sont identiques ou différents et représentent des atomes d'hydrogène ou des groupes alkyle en C₁-C₆,
3. un composé hétéroaromatique pris dans le groupe 3.1. à 3.15. suivant, qui est non substitué ou substitué comme décrit aux points 2.1. à 2.14.,
3.1. pyrrole,
3.2. pyrazole,
3.3. imidazole,
3.4. triazole,
3.5. thiophène,
3.6. thiazole,
3.7. oxazole,
3.8. isoxazole,
3.9. pyridine,
3.10. pyrimidine,
3.11. indole,
3.12. benzothiophène,
3.13. benzimidazole,
3.14. benzoxazole ou
3.15. benzothiazole,
4. -OH et A représente une liaison covalente,
5. -O-R¹⁴ et A représente une liaison covalente, -CH=CH- ou -C≡C- et où R¹⁴ représente
1) alkyle en C₁-C₆,
2) cycloalkyle en C₃-C₆,
3) benzyle ou
4) phényle,
6. -COOH et A représente une liaison covalente, -CH=CH- ou -C≡C-,
7. alkyle en C₁-C₆,
8. (cycloalkyle en C₃-C₆)-O-alkyle en C₁-C₄,
9. halogène et A représente une liaison covalente, -CH=CH- ou -C≡C-,
10. -CN et A représente une liaison covalente, -CH=CH- ou -C≡C-,
11. -NO₂ et A représente une liaison covalente, -CH=CH- ou -C≡C-, ou
12. -CF₃ et
R² représente un groupe
1. HO(H)N- ou
2. R⁷-O-, où R⁷ représente
2.1. un atome d'hydrogène
2.2. alkyle en C₁-C₆,
2.3. allyle ou
2.4. benzyle,
R³ et R⁴ sont identiques ou différents et représentent
1. un atome d'hydrogène,
2. alkyle en C₁-C₆,
3. phényl-(CH₂)ₘ, où phényle est non substitué ou substitué une ou deux fois comme décrit aux points 2.1. à 2.14. et m est un entier zéro, 1, 2 ou 3,
4. R⁸-(CO)-, où R⁸ représente
4.1. alkyle en C₁-C₈,
4.2. phényl-(CH₂)ₘ, où phényle est non substitué ou substitué une ou deux fois comme décrit aux points 2.1. à 2.14. et m est un entier zéro, 1, 2 ou 3,
4.3. R⁷-O-C(O) - (CH₂)ₙ-, où R⁷ est défini comme ci-dessus et n est un entier zéro, 1, 2, 3, 4, 5 ou 6,
4.4. R⁷-N(H)-(R⁹)-C(H)-, où R⁷ est défini comme ci-dessus et R⁹ représente le reste caractéristique d'un acide α-aminé protéinogène et où R⁸ est non substitué ou substitué sur un atome d'oxygène ou de soufre une ou deux fois par des groupes alkyle en C₁-C₄, benzyle, allyle ou est substitué par un groupe protecteur de N,
4.5. R⁷-O-C(O)-N(H)(R⁹)-C(H)-, où R⁷ et R⁹ sont définis comme au point 4.4., ou
4.6. R¹⁰-O-C(O)-N(H) (R⁹)-C(H)-, R⁹ étant défini comme aux points 4.4. et où R¹⁰ représente
4.6.1. alkyle en C₁-C₆,
4.6.2. allyle,
4.6.3. benzyle ou
4.6.4. (9-fluorényl)-méthyle,
5. R¹⁰-O-C(O)-, où R¹⁰ est défini comme aux points 4.6.1. à 4.6.4.,
6. R¹⁵-SO₂-, où R¹⁵ représente
6.1. alkyle en C₁-C₆,
6.2. allyle ou
6.3. phényl-(CH₂)ₘ, où phényle est non substitué ou substitué une ou deux fois comme décrit aux points 2.1. à 2.14. et m est un entier zéro, 1, 2 ou 3, ou
7. H₂N-C(=NH)-, ou
R³ et R⁴ forment ensemble avec l'atome de N un reste de formule Xₐ ou X_{b}, 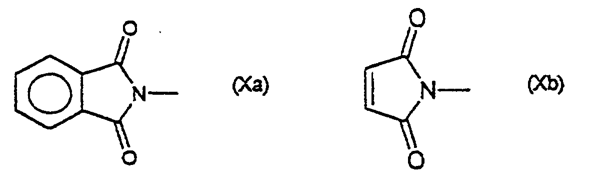 A représente
a) une liaison covalente,
b) -O-,
c) -CH=CH- ou
d) -C≡C-,
B représente
a) -(CH₂)ₘ-, où m possède la signification donnée ci-dessus,
b) -O-(CH₂)_{q}, où q est un entier 1, 2, 3, 4 ou 5, ou
c) représente -CH=CH- et
X représente un groupe -CH=CH-, un atome d'oxygène ou un atome de soufre.

2. Procédé pour la préparation du composé de formule I selon la revendication 1, **caractérisé en ce qu'**on transforme
a) le composé de formule II 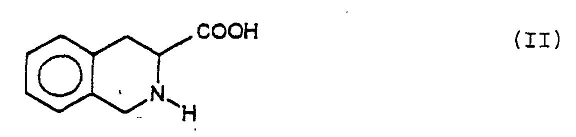 en un composé de formule III, 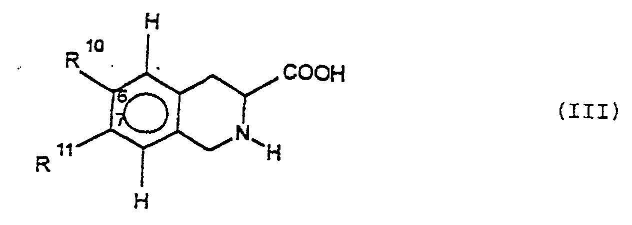 où R¹⁰ et R¹¹ représentent un groupe -NO₂ ou un atome d'hydrogène et R¹⁰ et R¹¹ ne sont pas identiques, et
b) on fait réagir le composé de formule III obtenu au point a) avec le composé de formule IV 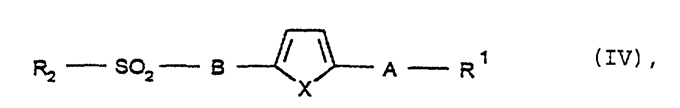 où B, X, A et R¹ sont définis comme à la formule I et R₂ représente un atome de chlore, un groupe imidazolyle ou -OH, en présence d'une base ou éventuellement d'un agent déshydratant, pour obtenir un composé de formule V 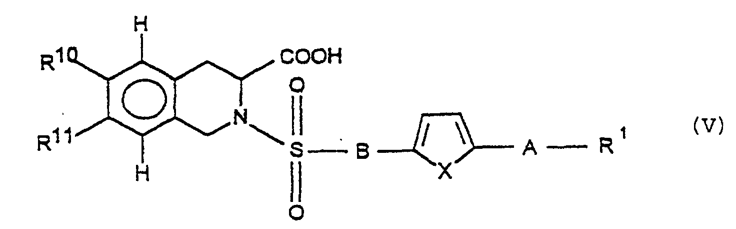 où R¹⁰ et R¹¹ représentent un groupe -NO₂ ou un atome d'hydrogène et R¹⁰ et R¹¹ ne sont pas identiques, et
c) on soumet le composé de formule V obtenu au point b) à une séparation d'isomères et on obtient un composé de formule I, où R³ et R⁴ forment, ensemble avec l'atome de N, un reste NO₂ qui est lié en position 6 ou 7 au reste phényle, ou
d) on réduit le composé obtenu au point c) en un composé de formule I, où R³ et R⁴ représentent un atome d'hydrogène, ou
e) on acyle un composé obtenu au point d) avec des chlorures d'acide carboxylique ou d'acide sulfonique, des imidazolides d'acide carboxylique ou d'acide sulfonique, des esters de l'acide formique, des esters actifs ou anhydrides, ou
f) on fait réagir un composé obtenu au point d) avec l'acide carboxylique, l'aldéhyde, l'aminoacide correspondants ou une guanidine éventuellement substituée, ou
g) on alkyle un composé obtenu au point d), ou
h) on fait réagir un composé obtenu au point a) pour obtenir un composé de formule VI 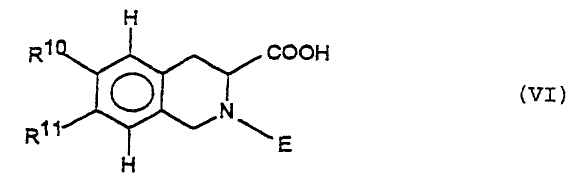 où E représente un groupe protecteur de N et R¹⁰ et R¹¹ sont définis comme ci-dessus et on sépare le composé de formule VI en les régioisomères de formule VII et VIII 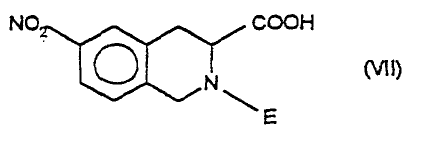 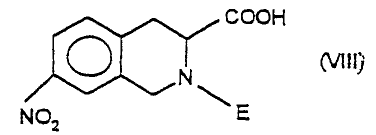 et on fait réagir le groupe nitro comme décrit au point d) et on fait réagir le composé comme décrit aux points e), f) ou g),
i) on fait réagir un composé obtenu selon les procédés h) , g) , f), e) ou d) en les esters d'acides carboxyliques correspondants (R² = O-R⁷) ou avec l'hydroxylamine (R² = -N(H)-OH).

3. Médicament **caractérisé par** une teneur efficace en au moins un composé de formule I selon la revendication 1 conjointement avec un véhicule approprié sur le plan pharmaceutique et toléré sur le plan physiologique, une matière d'addition et/ou d'autres substances active ou adjuvants.

4. Utilisation d'au moins un composé de formule I selon la revendication 1 pour la préparation de médicaments pour la prévention et la thérapie de maladies auxquelles participent des métalloprotéinases dégradant la matrice avec une activité augmentée.

5. Utilisation selon la revendication 4 pour le traitement de maladies d'articulations ankylosantes telles que des ostéoarthroses, des spondyloses, l'atrophie du cartilage, après un traumatisme de l'articulation ou une immobilisation prolongée de l'articulation suite à des blessures du ménisque ou de la rotule ou des déchirures de ligaments, de maladies de la trame conjonctive comme des collagénoses, des maladies périodontales, des troubles de cicatrisation et des maladies chroniques liées à la mobilité, telles que des arthropathies, des myalgies, des arthrites inflammatoires aiguës et chroniques provoquées par des troubles du système immunologique ou de métabolisme et des troubles de métabolisme osseux, d'ulcération, d'athérosclérose et de sténoses, mais aussi pour le traitement d'inflammations, de maladies cancéreuses, de formation de métastases cancéreuses, de cachexie, d'anorexie et de choc septique.

6. Procédé pour la préparation d'un médicament, **caractérisé en ce qu'**on met en une forme d'administration appropriée au moins un composé de formule I selon la revendication 1 à l'aide d'un véhicule approprié sur le plan pharmaceutique et toléré sur le plan physiologique et éventuellement à l'aide d'autres substances actives, de matières d'addition ou d'adjuvants appropriés.
